(19) Europäisches Patentamt
European Patent Office
Office européen des brevets

(11) Veröffentlichungsnummer : **0 066 248**
**B1**

(12) **EUROPÄISCHE PATENTSCHRIFT**

(45) Veröffentlichungstag der Patentschrift : **28.08.85**

(51) Int. Cl.⁴ : **C 07 D263/58**

(21) Anmeldenummer : **82104581.2**

(22) Anmeldetag : **26.05.82**

# (54) Verfahren zur Herstellung von Halogen-2-merkaptobenzoxazolen.

(30) Priorität : **01.06.81 DE 3121675**

(43) Veröffentlichungstag der Anmeldung : **08.12.82 Patentblatt 82/49**

(45) Bekanntmachung des Hinweises auf die Patenterteilung : **28.08.85 Patentblatt 85/35**

(84) Benannte Vertragsstaaten : **BE CH DE FR GB IT LI NL**

(56) Entgegenhaltungen :
**ORGANIC SYNTHESES, Band 30, 1950, Seiten 56,57, New York, USA J.A. VAN ALLAN et al.: "2-Mercaptobenzimidazole"**
**JOURNAL OF ORGANIC CHEMISTRY, Band 19, Nr. 5, Mai 1954, Seiten 758-766, Columbus, Ohio, USA L. KATZ et al.: "Benzoxazole derivatives. I. 2-Mercaptobenzoxazoles"**

(73) Patentinhaber : **HOECHST AKTIENGESELLSCHAFT**
**Postfach 80 03 20**
**D-6230 Frankfurt am Main 80 (DE)**

(72) Erfinder : **Handte, Reinhard, Dr.**
**Breckenheimer Strasse 45**
**D-6238 Hofheim am Taunus (DE)**
Erfinder : **Sander, Jürgen, Dr.**
**Berliner Ring 27**
**D-6233 Kelkheim(Taunus) (DE)**
Erfinder : **Tammer, Thomas, Dr.**
**Höchster Strasse 22**
**D-6238 Hofheim am Taunus (DE)**

**EP 0 066 248 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung ist ein Verfahren zur Herstellung von Halogen-2-merkaptobenzoxazolen der allgemeinen Formel I

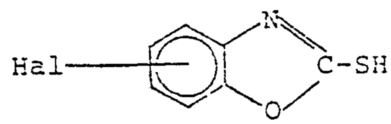

Darin steht « Hal » für Halogen in 5- oder 6-Stellung, insbesondere für Fluor, Chlor und Brom.

2-Merkaptobenzoxazol wurde erstmals von Dünner (Ber. *9* (1876) 465) beschrieben. In der Folgezeit beschäftigten sich verschiedene Autoren mit der Herstellung und den Umsetzungen von (ggf. substituierten) 2-Merkaptobenzoxazolen (z. B. Ber. *16* (1883) 1825 ; JACS *55* (1933) 4989 ; J. chem. Soc. *1934*, 1186 ; JACS *75* (1952) 2770-71 ; J. org. Chem. *19* (1954), 758 ; Ber. *89* (1956), 1014 und Org. Synth. *30*, 56).

2-Merkaptobenzoxazole und insbesondere die 5- und 6-Halogen-derivate waren bislang nicht Gegenstand größeren wissenschaftlichen oder technischen Interesses. Die meisten bislang bekanntgewordenen Herstellungsverfahren liefern die 2-Merkaptobenzoxazole nur in mäßigen bis schlechten Ausbeuten bzw. haben den Nachteil, daß bei erhöhten Temperaturen mit Schwefelkohlenstoff gearbeitet werden muß. Katz und Cohen (J. org. Chem. *19* (1954) 758) konnten erstmals zeigen, daß es möglich ist, 2-Merkaptobenzoxazole durch Umsetzung von frisch aus Methanol, Kaliumhydroxid und Schwefelkohlenstoff hergestelltem Kaliummethylxanthat mit substituierten 2-Aminophenolen auch in guten Ausbeuten herzustellen. 6-Chlor-2-merkaptobenzoxazol läßt sich nach Angaben der Autoren jedoch nur in einer äußerst unbefriedigenden Ausbeute von 48 % der Theorie herstellen, für das entsprechende 5-Chlorderivat fehlt jede Ausbeuteangabe. Dieser schlechten Herstellbarkeit der 5- und 6-Halogen-2-merkaptobenzoxazole steht neuerdings ein gesteigertes Interesse an diesen Verbindungen als Ausgangsmaterialien zur Herstellung wertvoller Zwischen- und Endprodukte gegenüber. So lassen sich diese beispielsweise durch Chlorierung zu den entsprechenden 2-Chlorbenzoxazolen oder durch Alkylierung und Oxidation zu den entsprechenden 2-Alkylsulfonyl- oder -sulfinylbenzoxazolen umsetzen, die ihrerseits gemäß DE-OS 26 40 730 oder EP 18 080 hervorragend wirksame Herbizide liefern. Es bestand daher ein Bedürfnis nach einer wirtschaftlichen, vom Sicherheitsstandpunkt unbedenklichen und unter ökologischen Aspekten vertretbaren technischen Synthese der 5- und 6-Halogen-2-merkaptobenzoxazole, bei der die genannten Probleme nicht auftreten. Diese Aufgabe wird durch das erfindungsgemäße Verfahren gelöst, welches dadurch gekennzeichnet ist, daß man die entsprechenden 4- oder 5-Halogen-2-aminophenole mit Alkaliniederalkylxanthaten in heterogener Phase umsetzt.

Den bekannten Verfahren zur Herstellung von (substituierten) Merkaptobenzoxazolen aus 2-Aminophenolen und Xanthaten ist gemeinsam, daß die Reaktion in Lösung durchgeführt wird, wobei in der Regel Ethanol, ggf. mit einem geringen Anteil (< 20 %) Wasser, als Lösungsmittel verwendet wird. Es ist daher überraschend, daß für die Erzielung hoher Ausbeuten insbesondere solche Lösungsmittel oder Lösungsmittelgemische geeignet sind, in denen einer der beiden Reaktionspartner sowie ggf. auch das primär als Endprodukt entstehende Merkaptid nicht oder nur sehr wenig löslich sind.

Als Lösungsmittel für das erfindungsgemäße Verfahren eignen sich aprotische Lösungsmittel, insbesondere aromatische Kohlenwasserstoffe wie z. B. Xylol, Chlor- oder Dichlorbenzol. Außer diesen eignet sich für die Reaktion auch Wasser, was infolge der relativen Instabilität der Xanthate in Wasser bei höheren Temperaturen besonders überraschend ist.

Für den Ringschluß mit dem Chloraminophenol kommen frisch (aus Alkohol, wäßrigem Alkali und $CS_2$) zubereitete Xanthate ebenso wie technische Xanthate in Frage. Letztere insbesondere Natrium- und Kalium(methyl bis hexyl)-xanthate, sind Grundchemikalien, die in großem Umfang als Flotationshilfsmittel Verwendung finden. Die Reaktion wird in der Regel bei Temperaturen oberhalb 50 °C, zweckmäßigerweise oberhalb 80 °C durchgeführt, ggf. kann auch im geschlossenen System gearbeitet werden. Der während der Reaktion gebildete Schwefelwasserstoff wird kontinuierlich durch Abgasen aus dem Reaktionsmedium entfernt und extern absorbiert. Unter den handelsüblichen Xanthaten, die vorteilhaft in die Reaktion eingesetzt werden, seien im einzelnen Natrium- und Kaliumethylxanthat, Natrium- und Kaliumpropyl und -isopropyl- sowie Natrium- und Kaliumisobutylxanthat genannt.

Die nachfolgenden Beispiele sollen das erfindungsgemäße Verfahren näher erläutern, ohne daß dadurch eine Einschränkung beabsichtigt ist.

Beispiel 1

6-Chlor-2-merkaptobenzoxazol

In einem 1 Liter-Rührkolben mit Rührer, Thermometer und Rückflußkühler werden 143,6 g (1 mol) 5-Chlor-2-aminophenol, 164 g (1,02 mol) Natriumethylxanthat (90 %-ig) und 500 ml Wasser eingetragen. Das Reaktionsgemisch (das Chloraminophenol bleibt ungelöst) wird unter Rühren erwärmt, ab 80 °C setzt

eine heftige Schwefelwasserstoffentwicklung ein. Nach ca. einer Stunde ist die Reaktion weitgehend abgeschlossen, zur Vervollständigung der Umsetzung werden weitere zwei Stunden unter Rückfluß nachgerührt. Die resultierende schwarze Reaktionslösung wird abgekühlt und mit 70 g einer 85 %-igen Phosphorsäure sauer gestellt. Der ausgefallene Niederschlag wird abgesaugt, mit Wasser gründlich gewaschen und anschließend bei ca. 80 °C im Vakuum getrocknet. Nach Trocknen erhält man 180,5 g (97,3 % d. Th.) an 6-Chlor-2-merkaptobenzoxazol mit einem Festpunkt von 221-223 °C.

(Literatur : Fp. 224-225 nach Umkristallisieren aus Wasser/Isopropanol, J. org. Chem. *19,* 758).

Die Reinheit des Produktes beträgt nach gaschromatographischer Bestimmung > 98 %.

Beispiel 2

6-Chlor-2-merkaptobenzoxazol

Es wird analog der Arbeitsweise des Herstellungsbeispiels 1 gearbeitet, anstelle von Natriumethylxanthat werden jedoch 213,1 g (1,02 mol) Kaliumisobutylxanthat eingesetzt. Nach Aufarbeitung werden 178 g (96 % d. Th.) an 6-Chlor-2-merkaptobenzoxazol mit einem Festpunkt von 222-223 °C erhalten, Reinhait nach GC > 98 %.

Beispiel 3

5-Chlor-2-merkaptobenzoxazol

Unter Verwendung der in Beispiels 1 beschriebenen Apparatur werden 143,6 g (1 mol) 4-Chlor-2-aminophenol und 181,3 g (1,02 mol) Kaliumethylxanthat (90 %-ig) in 500 ml Wasser unter Rückflußtemperatur umgesetzt. Die Reaktion wird ebenfalls innerhalb von drei Stunden durchgeführt. Die dunkle Reaktionslösung wird mit konzentrierter Salzsäure angesäuert, der Produktniederschlag wird abgesaugt und mit Wasser neutral gewaschen. Nach Trocknen im Vakuum bei 80 °C erhält man 179 g (96,7 % d. Th.) an 5-Chlor-2-merkaptobenzoxazol mit einem Festpunkt von 269-271 °C (Literatur Fp. 260-261 °C J. org. Chem. *19,* 758).

Beispiel 4

6-Chlor-2-merkaptobenzoxazol bzw. dessen Natriumsalz

Analog Beispiel 1 werden 143,6 g (1 mol) 5-Chlor-2-aminophenol und 164 g (1,02 mol) Natriumethylxanthat in 600 ml 1,2-Dichlorbenzol unter Heizen und Rühren umgesetzt. Ab 80-82 °C Innentemperatur setzt eine lebhafte Schwefelwasserstoffentwicklung ein, gleichzeitig fällt gebildetes Natriummerkaptid aus. Nach 5 Stunden wird der Ansatz andestilliert, abgekühlt, abgesaugt und bei 80 °C im Vakuum getrocknet, es resultieren 209 g eines Feststoffes, der fast auschließlich aus dem Natriumsalz von 6-Chlor-2-merkaptobenzoxazol besteht. Zum Erhalten der freien Merkaptoverbindung wird das Salz in 1 200 ml Wasser gelöst, anschließend wird mit 2 n-Schwefelsäure auf pH 1 gestellt, der gebildete Feststoff wird abgesaugt, mit Wasser gründlich nachgewaschen und bei 80 °C im Vakuum getrocknet. Nach dem Trocknen erhält man 178 g (96 % d. Th.) an 6-Chlor-2-merkaptobenzoxazol mit einem Festpunkt von 223-225 °C.

Beispiel 5

Es wird wie in Beispiel 4 verfahren, anstelle von 1,2-Dichlorbenzol wird jedoch Chlorbenzol verwendet. Als Xanthat werden 181,3 g (1,02 mol) Kaliumethylxanthat eingesetzt. Nach einer Reaktionszeit von fünf Stunden bei 90 °C wird andestilliert, abgekühlt und abgesaugt. Das Produkt wird mit 500 ml Chlorbenzol nachgewaschen und im Vakuum bei 80 °C getrocknet. Nach Trocknen erhält man 226 g eines Feststoffes, der fast ausschließlich aus dem Kaliumsalz von 6-Chlor-2-merkaptobenzoxazol besteht. Die Freisetzung der Merkaptoverbindung erfolgt wie in Beispiel 4. Nach Trocknen erhält man 179 g (96,5 % d. Th.) an 6-Chlor-2-merkaptobenzoxazol mit einem Festpunkt von 222-224 °C.

Gewünschtenfalls kann das primär anfallende Kaliummerkaptid aber auch direkt weiter umgesetzt werden, z. B. mit Chlor.

**Patentansprüche**

1. Verfahren zur Herstellung von 5- und 6-Halogen-2-merkaptobenzoxazolen bzw. ihrer Natrium- oder Kaliumsalze, dadurch gekennzeichnet, daß man die entsprechenden 4- oder 5-Halogen-2-aminophenole mit Natrium- oder Kalium (-niederalkyl)xanthaten in heterogener Phase umsetzt.

2. Verfahren nach Anspruch 1, dadurch gekennzeichnet, daß die Umsetzung in einem aromatischen Kohlenwasserstoff durchgeführt wird.

3. Verfahren nach Anspruch 1 oder 2, dadurch gekennzeichnet, daß die Umsetzung in Chlor- oder Dichlorbenzol durchgeführt wird.

4. Verfahren nach Anspruch 1, dadurch gekennzeichnet, daß die Umsetzung in wäßriger Suspension durchgeführt wird.

5. Verfahren nach einem der Ansprüche 1 bis 4, dadurch gekennzeichnet, daß zur Umsetzung technische Xanthate eingesetzt werden.

**Claims**

1. A process for the preparation of 5- and 6-halo-2-mercaptobenzoxazoles or the sodium and potassium salts thereof, which comprises reacting corresponding 4- or 5-halo-2-aminophenols with sodium or potassium lower alkyl xanthates in heterogeneous phase.

2. The process as claimed in claim 1, which comprises carrying out the reaction in an aromatic hydrocarbon.

3. The process' as claimed in claim 1 or 2, which comprises carrying out the reaction in chlorobenzene or dichlorobenzene.

4. The process as claimed in claim 1, which comprises carrying out the reaction in aqueous suspension.

5. The process as claimed in any of claims 1 to 4, which comprises using industrial-grade-xanthates as starting materials for the reaction.

**Revendications**

1. Procédé pour préparer des halogéno-5 et des halogéno-6 mercapto-2 benzoxazoles ou leurs sels sodiques ou potassiques, procédé caractérisé en ce qu'on fait réagir les halogéno-4 ou halogéno-5 amino-2 phénols correspondants avec des alkyl-xanthates de sodium ou de potassium à alkyle inférieur, en phase hétérogène.

2. Procédé selon la revendication 1 caractérisé en ce qu'on effectue la réaction dans un hydrocarbure aromatique.

3. Procédé selon l'une des revendications 1 et 2, caractérisé en ce qu'on effectue la réaction dans du chlorobenzène ou dans un dichlorobenzène.

4. Procédé selon la revendication 1 caractérisé en ce qu'on effectue la réaction en suspension aqueuse.

5. Procédé selon l'une quelconque des revendications 1 à 4, caractérisé en ce qu'on utilise, pour la réaction, des xanthates techniques.